Europäisches Patentamt

European Patent Office

Office européen des brevets

(11) Publication number: **0 378 983**
**A1**

## (12) EUROPEAN PATENT APPLICATION

(21) Application number: **89870156.0**

(22) Date of filing: **20.10.89**

(51) Int. Cl.⁵: **A61F 5/01, A61F 13/04**

The title of the invention has been amended (Guidelines for Examination in the EPO, A-III, 7.3).

(30) Priority: **24.11.88 BE 8801333**

(43) Date of publication of application:
**25.07.90 Bulletin 90/30**

(84) Designated Contracting States:
**AT CH ES FR GB IT LI SE**

(71) Applicant: **Dierick, Mathieu**
**Industrieweg 74**
**BE-3980 Tessenderlo(BE)**

(72) Inventor: **Dierick, Mathieu**
**Industrieweg 74**
**BE-3980 Tessenderlo(BE)**

(74) Representative: **Donné, Eddy**
**Bureau M.F.J. Bockstael nv Arenbergstraat 13**
**B-2000 Antwerpen(BE)**

(54) Belt attachment for an orthopaedic sole.

(57) Belt attachment for an orthopaedic sole, characterised in that it principally consists of two belt parts (4, 5) which are respectively, directly or indirectly, connected to opposite sides (6, 7) of the sole (1), and an intermediate piece (8) that can connect both belt parts (4, 5) to each other, such that one belt part (4) passes freely through the intermediate piece (8), while the other belt part (5) can be clamped and/or stretched through the intermediate piece (8) so that the length of the belt attachment (2, 3) is adjustable, whereby the former belt part (4) shows two parts (9, 10) of a fastener (11) which can work together by folding double this belt part (4).

Fig.1

EP 0 378 983 A1

## Belt attachment for an orthopaedic sole and orthopaedic sole which utilises such belt attachment.

This invention relates to a belt attachment for an orthopaedic sole, as well as for orthopaedic soles which utilise such belt attachment.

It is known that a support in the form of a stump is installed under a plaster cast of a foot, such to render a rolling step movement of the foot possible without damaging the plaster cast. Such stump shaped support however shows the disadvantage that this offers an unstable bearing surface, that the plaster cast becomes dirty, and that the patient has difficulty in driving a car with it.

A known solution for the aforementioned disadvantages consists in the so-called walking sole which shows a special walking surface such that an easy rolling step movement of the foot is possible. Such orthopaedic sole is installed under the complete underside of the foot, respectively of the plaster cast and is held in place by means of belt attachments.

With the known orthopaedic soles such belt attachment is provided with a classic buckle. Such a fastener shows the disadvantage that the length of the belt attachment can only be adjusted over a limited distance and an orthopaedic sole with a belt attachment of another length must be taken each time the sole must be installed either under a synthetic cast that as known is very thin, or under a classic plaster cast that is relatively thick.

The purpose of the present invention is a belt attachment for an orthopaedic sole whereby the aforementioned disadvantage is excluded. Thus the belt attachment according to the invention shows the advantage that the same belt attachment can be applied for both a synthetic and a classic plaster cast. Another advantage of the belt attachment according to the invention consists in that it can be released and fastened simply.

For this purpose the present invention concerns a belt attachment for an orthopaedic sole with the characteristic that it consists of two belt parts which are respectively, directly or indirectly, connected to opposite sides of the sole and an intermediate piece that can connect both belt parts to each other, whereby one belt part passes freely through the intermediate piece, while the other belt part can be clamped and/or stretched through the intermediate piece such that the length of the belt attachment is adjustable, whereby the former belt part shows two parts of a fastener which can work together by folding double this belt part. In the preferred embodiment the intermediate piece consists of a sliding buckle which can be moved along the latter belt part, while the aforementioned fastener consists of a Velcro fastener.

It is clear that the use of the Velcro fastener permits the simple release and refastening of the belt attachment, whereby irrespective of the adjusted length of the belt attachment this Velcro fastener can always be closed over its complete length.

In order to show better the characteristics according to the present invention, a preferred embodiment is described hereafter, as example and without any restrictive character, with reference to the enclosed drawings, in which:

figure 1 shows an orthopaedic sole which utilises the belt attachment according to the invention;

figure 2 shows in opened position the belt part that includes the Velcro fastener;

figure 3 shows a buckle with which both belt parts can be joined to each other;

figure 4 shows a view according to arrow F4 in figure 1, more especially in the case that the belt attachment is installed around a thin cast;

figure 5 shows a similar view to that of figure 4, but in the case that the same belt attachment is installed around a thick cast.

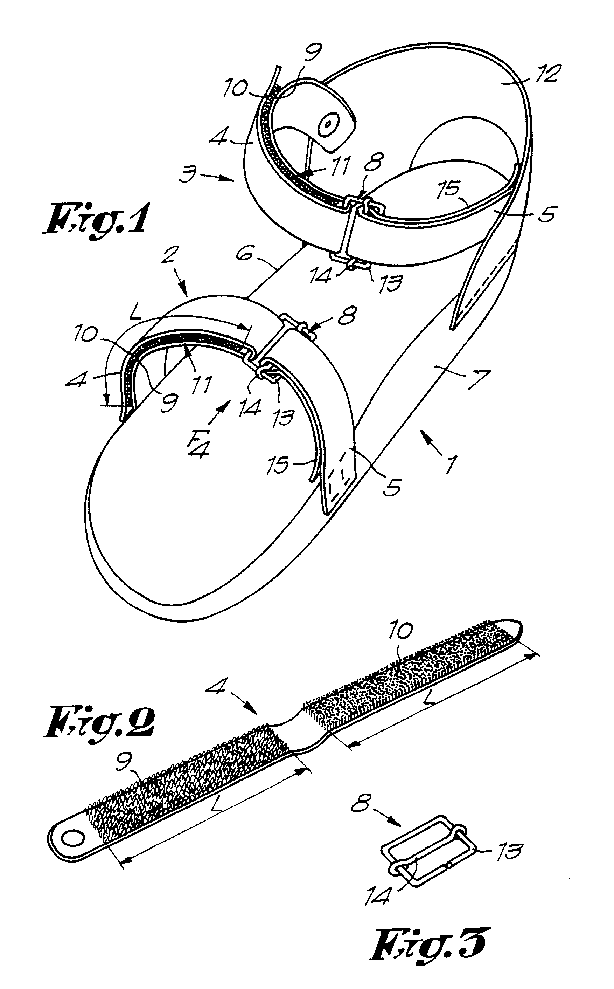

In figure 1 an orthopaedic sole 1 is shown which includes two belt attachments 2 and 3 according to the invention. These belt attachments 2 and 3 each consist of two belt parts 4 and 5 which are respectively joined to the opposite sides 6 and 7 of the sole 1 and an intermediate piece 8 that can join the two belt parts 4 and 5 to each other, whereby, on the one hand, the first belt part 4 passes freely through the intermediate piece 8 and shows two parts 9 and 10 of a fastener, for example a Velcro fastener 11, which can work together by folding double the belt part 4, and on the other hand, the second belt part 5 is clamped through the intermediate piece 8, such that the length of the belt attachment 2 or 3 in question is adjustable.

It is clear that the belt parts can be attached both directly and indirectly to the sole 1. This is clarified in figure 1 whereby the front belt attachment 2 is attached directly to the sole 1, while the back belt attachment 3 is connected to a support 12.

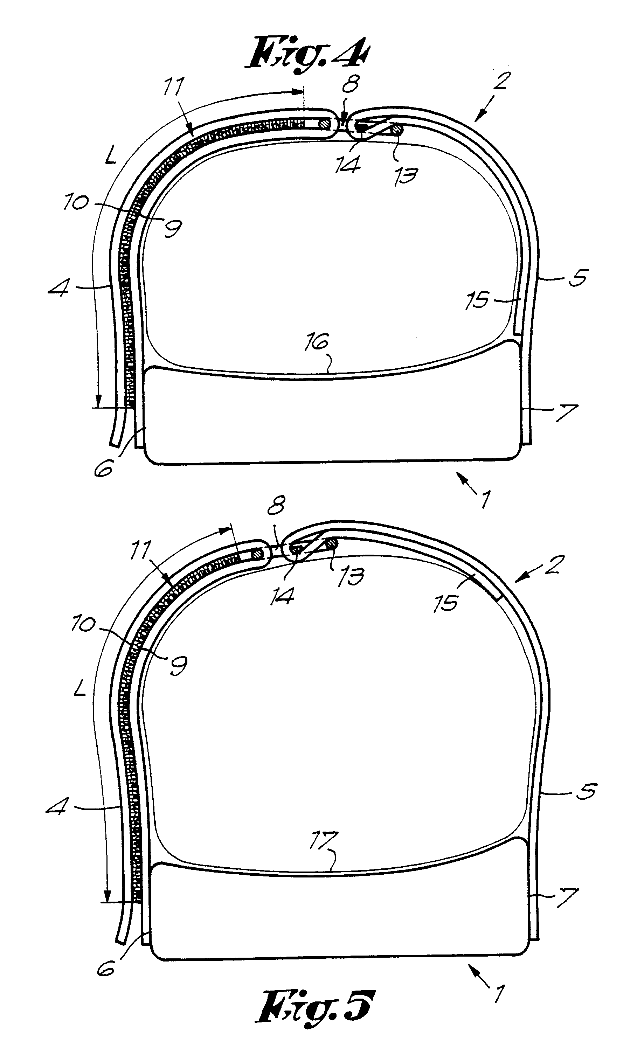

As shown in figures 1 and 2 the aforementioned parts 9 and 10 extend over almost half the length of the belt part 4, such that the length of the belt part 4 is optimally utilised in order to obtain a Velcro fastener 11 with as great a fastening surface as possible.

As shown in figure 3 the intermediate piece 8 preferably consists of a sliding buckle formed by a square bracket 13 with a sliding transverse connection 14. As shown in figure 1 the belt part 5 is guided with its free extremity 15 around the transverse connection 14 and through the bracket 13.

When installing the orthopaedic sole under a plaster cast the Velcro fastener 11 is released and the belt part 4 is pushed out of the bracket 13. Subsequently the sliding buckle 8 is adjusted in relation to the belt part 5 by sliding this and by such that when reinstalling the belt part 4 the Velcro fastener 11 can be closed over its maximal length L.

In figures 4 and 5 the use of the belt attachment according to the invention is respectively shown around a thin cast 16 and a thick cast 17, for example, on the one hand a synthetic cast and on the other hand a classic plaster cast. From these figures it is clear that through the adjustment of the position of the intermediate piece 8 in relation to the belt part 5 the alteration of the belt attachment is possible over a great length without altering the length L over which the Velcro fastener 11 closes, so that a maximal fastening is constantly maintained. The belt parts 4 and 5 preferably consist of leather while the parts 9 and 10 of the Velcro fastener are sewn onto these.

The invention also relates to an orthopaedic sole, with the characteristic that this shows at least one belt attachment as described for this purpose. In a preferred embodiment use is made of two belt attachments 2 and 3 which as shown in figure 1 and as described above are attached to the sole 1 respectively at the front and at the back.

The present invention is in no way restricted to the embodiment described as example and shown in the drawings, but such belt attachment for an orthopaedic sole, and a sole which utilises such attachment, may be developed in all kinds of forms and dimensions without departing from the scope of the present invention

## Claims

1.- Belt attachment for an orthopaedic sole, characterised in that it principally consists of two belt parts (4, 5) which are respectively, directly or indirectly, connected to opposite sides (6, 7) of the sole (1), and an intermediate piece (8) that can connect both belt parts (4, 5) to each other, such that one belt part (4) passes freely through the intermediate piece (8), while the other belt part (5) can be clamped and/or stretched through the intermediate piece (8) so that the length of the belt attachment (2, 3) is adjustable, whereby the former belt part (4) shows two parts (9, 10) of a fastener (11) which can work together by folding double this belt part (4).

2.- Belt attachment according to claim 1, characterised in that the aforementioned fastener (11) consists of a Velcro fastener.

3.- Belt attachment according to claim 1 or 2, characterised in that the intermediate piece (8) consists of a sliding buckle.

4.- Belt attachment according to claim 3, characterised in that the sliding buckle (8) consists of a square bracket (13) and a sliding transverse connection (14), whereby the clamped belt part is folded around the transverse connection (14) and through the bracket (13).

5.- Belt attachment according to one of the claims 2, 3 or 4, characterised in that both parts of the Velcro fastener (9, 10) each respectively cover almost half of the belt part (4).

6. - Belt attachment according to one of the claims 2 through 5, characterised in that the belt parts (4, 5) consist of leather, while the parts (9, 10) of the Velcro fastener (11) are sewn onto these.

7.- Orthopaedic sole, characterised in that it utilises at least one belt attachment according to one of the aforementioned claims.

8.- Orthopaedic sole according to claim 7, characterised in that it includes at least two belt attachments (2, 3) respectively at the front and at the back of the sole (1).

9.- Orthopaedic sole according to claim 8, characterised in that the front belt attachment (2) is attached directly to the sole (1) while the back belt attachment (3) is installed on a support (12) mounted on the sole (1).

*Fig.1*

*Fig.2*

*Fig.3*

EP 0 378 983 A1

*Fig.4*

*Fig.5*

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | DE-A-2 733 357 (BIEDERMANN) <br> * Figures; page 9, line 16 - page 10, line 15 * <br> --- | 1 | A 61 F 5/01 <br> A 61 F 13/04 |
| A | US-A-4 351 324 (BRONKHORST) <br> --- | | |
| A | EP-A-0 060 758 (HERMES PARIS) <br> ----- | | |
| | | | TECHNICAL FIELDS SEARCHED (Int. Cl.5) |
| | | | A 61 F <br> A 43 B <br> A 43 C |

The present search report has been drawn up for all claims

| Place of search | Date of completion of the search | Examiner |
|---|---|---|
| THE HAGUE | 01-03-1990 | STEENBAKKER J. |